# EUROPEAN PATENT APPLICATION

(11) **EP 3 932 357 A1**
(43) Date of publication of application: **05.01.2022**
(21) Application number: 20183380.3
(22) Date of filing: 01.07.2020
(51) Int. Cl.: A61B 34/10, A61B 34/20, A61B 90/00, A61B 17/00, A61B 17/70, A61B 34/00

(54) **SYSTEM FOR ASSISTING A USER IN PLACING A PENETRATING DEVICE IN TISSUE**

(71) Applicant: Koninklijke Philips N.V., 5656 AG Eindhoven (NL)
(72) Inventor: SPLIETHOFF, Jarich Willem, 5656 AE Eindhoven (NL); HENDRIKS, Bernardus Hendrikus Wilhelmus, 5656 AE Eindhoven (NL); BABIC, Drazenko, 5656 AE Eindhoven (NL); GROEN, Johanneke Gerrigje, 5656 AE Eindhoven (NL); REICH, Christian, 5656 AE Eindhoven (NL); HOLTHUIZEN, Ronaldus Frederik Johannes, 5656 AE Eindhoven (NL); HOMAN, Robert Johannes Frederik, 5656 AE Eindhoven (NL)
(74) Representative: Philips Intellectual Property & Standards

(57) **Abstract**

The invention relates to a system 8 for assisting a user in placing a penetrating device in tissue like a pedicle screw 7 in a vertebra's pedicle. The system generates a virtual view 20 from a penetrating device tip perspective within the tissue in the direction of a path 21 through a model of the tissue. The virtual view is generated based on tracking information indicating a pose of the penetrating device, the model and the path, wherein the virtual view is configured such that it indicates a direction in which the user should move the penetrating device while placing it in the tissue. For instance, it can show a virtual tunnel 801 which is arranged along the path. If a user like a surgeon is provided with such a virtual view, the user can position the penetrating device along the path with a significantly increased accuracy.

## Description

### FIELD OF THE INVENTION

The invention relates to a system, method, computer program and data carrier comprising the computer program for assisting a user in placing a penetrating device in tissue of a subject. The penetrating device may be a pedicle screw to be placed in a pedicle of a vertebra.

### BACKGROUND OF THE INVENTION

A known system for assisting a user in placing a penetrating device in tissue is, for instance, a system disclosed in WO 2017/055144 A1 for use during spinal fusion surgery. Pedicle screw placement is a critical step in spinal fusion surgery, i.e. it is challenging and poses risks as vital portions of the spinal anatomy in neurovascular structures are not visible to a surgeon. Traditionally, pedicle screws are placed freehand, wherein the surgeon relies on anatomical landmarks and preoperatively acquired images and wherein x-ray fluoroscopy can be used to provide guidance and confirm adequate screw placement. However, even if x-ray fluoroscopy is used, it is challenging for the surgeon to accurately position the pedicle screw in a vertebra.

### SUMMARY OF THE INVENTION

It is an object of the present invention to provide a system, a method , a computer program optionally comprised on a data carrier for assisting a user in placing a penetrating device in tissue like in a pedicle of a vertebra, which allow to position the penetrating device more accurately and/or with less risk of anatomical damage.

In a first aspect the claims define a system as defined in claim 1.

It has been found by the inventors that generating the virtual view from a perspective of a tip of the penetrating device within the tissue, such as within a pedicle of a vertebra, in the direction of the path of the penetrating device through the tissue and showing this virtual view to a medical practitioner (e.g. a surgeon) while placing the penetrating device in the tissue, allows to position the penetrating device along the path with a significantly increased accuracy. This may therefore improve the procedural outcome and/or reduce cost of the procedure, e.g because of time reduction and/or reduction of number of repeat procedures to correct error.

In the claims, the pose includes the position, i.e. location, and preferably also the orientation of the penetrating device, preferentially of a tip portion of the penetrating device. The path is a virtual path through the model of the tissue, which indicates a route through the tissue, along which the penetrating device should be moved.

The first, second and third inputs are configured to communicate the respective received data or data signals carrying such data to the processor. These inputs may be all separate from each other for example to receive the data/or signal parallel in time. Alternatively, or additionally, two or all three of these inputs may be combined in one and in such case the combined input may receive the data or signals at least partly in series.

In a preferred embodiment the processor is configured to generate the virtual view such that it shows a virtual tunnel within the tissue, wherein the virtual tunnel is arranged along the provided path such that the virtual view is a view in the virtual tunnel in the direction of the provided path. It is hereby noted that in real the tunnel is not there, because it is assumed that the tissue is a mass, particularly bone, without a tunnel. The tunnel is just a virtual tunnel which is used for guiding the user while placing the penetrating device. This allows to guide the user intuitively such that he/she places the penetrating device along the provided path, because the user tries to move the penetrating device such that he/she "walks" or moves through the tunnel. This further improves the assistance of the user while placing the penetrating device in the tissue.

The tissue is preferentially bone, particularly bone of a vertebra. The model is preferentially a model of the vertebra, which shows at least the bone part of the vertebra. Preferentially, the model providing unit is configured to provide the model such that it also shows further structures of the vertebra like the spinal cord and/or neighboring structures like a blood vessel. The model shows preferentially at least the cortical bone and can also show the cancellous bone.

In a preferred embodiment the tissue is bone and the hollow part of the virtual tunnel represents a bone type of a first density and the wall of the virtual tunnel represents a bone type of a second density, wherein the first density is smaller than the second density. In particular, the model can be provided such that it distinguishes between a first bone tissue type having a first density and a second bone tissue type having a second density, wherein the second bone tissue type at least partly encloses the first bone tissue type and wherein the virtual tunnel is generated such that the inner hollow part of the tunnel represents the first bone tissue type and the outer wall of the tunnel represents the second bone tissue type.

The penetrating device is preferentially a bone-penetrating device. In an embodiment it is a screw like a pedicle screw. Moreover, in an embodiment the penetrating device is a device for generating a hole in tissue like bone, i.e. a device which is just used for generating the hole and not used for being permanently implanted, like an awl, a pedicle probe, a k-wire, a drill, a tap, et cetera. The penetrating device can also be another surgical instrument being configured to penetrate the tissue, particularly the bone. The way the device penetrates the tissue depends on the type of the device. For instance, a pedicle screw, tape or drill are inserted by rotation in combination with downward pressure, whereas a k-wire might be hammered.

The path providing unit is preferentially configured to provide a path through a pedicle of the model of the vertebra.

The tracking information providing unit can be a receiving unit for receiving the tracking information from another device like a tracking device and to provide the received tracking information. The tracking information providing unit can also itself be the tracking device. The tracking device can be, for instance, an optical tracking device, an electromagnetic tracking device, et cetera.

The model providing unit can be configured to receive the three-dimensional model from another device and to provide the received three-dimensional model. This other device can be a storing unit in which the three-dimensional model is stored or it can be a device which is configured to actually generate the three-dimensional model, for instance, based on an image of the tissue, particularly of bone, and possibly surrounding structures like a three-dimensional medical image such as a computed tomography image, a magnetic resonance image, et cetera. The model providing unit itself can also be the device which is configured to generate the three-dimensional model.

Thus, the model providing unit can be configured to provide an image of the tissue and to segment the image of the tissue, in order to generate the model. The model providing unit can be configured to receive the image of the tissue from an imaging system like a computed tomography imaging system or another anatomical imaging system, to segment the received image of the tissue for generating the model and to provide the generated model. However, the model providing unit itself could also be configured to acquire an image and then to segment the acquired image, in order to generate the model.

The path providing unit can be configured to receive the path from another device and to provide the received path. This other device can be, for instance, a storing unit in which the path is stored or a device which is configured to generate the path. The path providing unit can also itself comprise the storing unit or be configured to generate the path and to provide the generated path. In the latter case the path providing unit is preferentially adapted to generate the path based on the provided three-dimensional model.

For generating the virtual view the tracking information, the provided model and the provided path are registered to each other. If the provided model and the provided path both have been generated based on a same image of the tissue or if the provided path has been generated based on the provided model, the provided model and the provided path are automatically registered to each other. Otherwise, they could be registered to each other by using known registration techniques. For registering the tracking information with the provided image and hence with the provided model and the provided path a tracking device can be registered with the image, for instance, by using markers which are identifiable in the image and trackable by the tracing device. However, also other known registration techniques are possible.

The path providing unit is preferentially configured to calculate the path at least based on the shape and dimensions of the tissue as defined by the model. In a preferred embodiment the tissue is the bone of a vertebra comprising a pedicle, wherein the model providing unit is configured to provide a three-dimensional model of the bone of the vertebra comprising the pedicle as the model and wherein the path providing unit is configured to calculate the path based on the shape and dimensions of the pedicle as provided by the model. In particular, the path providing unit is configured to map an hourglass-shaped model to the pedicle provided by the model and to calculate the path based on the mapped hourglass-shaped model. Moreover, the path providing unit can be configured to calculate the path further based on the orientation of end plates of the body of the bone of the vertebra as provided by the model, wherein the end plates are on top of and below the vertebra, when a person is standing. This allows to provide the path such that, if the penetrating device is accurately placed along this path, a cortical breach and a negative influence on neighboring structures do very likely not occur.

In an embodiment the processor is configured to generate a visualization indicating a geometrical relationship between a) a current pose of the penetrating device and b.1) a target pose of the penetrating device as defined by the path based on the tracking information and based on the provided path and/or b.2) provided poses of regions of interest within the tissue based on the tracking information and based on the provided poses of the regions of interest. Moreover, the processor can be configured to generate the visualization such that it includes an overlay of the geometrical relationship over the virtual view. This allows for a further improved assisting of the user in placing the penetrating device in the tissue, thereby further reducing the likelihood of, for instance, a cortical breach or a damage of surrounding structures.

The processor can be configured to determine a distance between the provided path and the position of the penetrating device as indicated by the tracking information based on the provided model and the provided tracking information and to generate a signal based on the determined distance. If the path is arranged along a central rotational axis of a pedicle, this corresponds to determining a distance between the central rotational axis of the pedicle and the position of the penetrating device as indicated by the tracking information based on the provided model and the provided tracking information and to generating a signal based on the determined distance. The distance could be defined as being the shortest distance between the tip of the penetrating device and the provided path. The signal could be a warning signal which is generated if the determined distance is larger than a predefined threshold. The signal could also be generated if the distance is smaller than a predefined threshold, in order to indicate that the user is "on the right track". The signal can be an acoustical and/or optical signal. This can further ensure that the penetrating device is placed along the path within the tissue and does not adversely affect surrounding structures like the spinal cord.

In a further preferred embodiment the processor is configured to determine a desired angle of entrance of the penetrating device into the tissue like the bone of a vertebra based on the provided path and the provided model, to determine a current angle of entrance of the penetrating device into the tissue based on the provided tracking information and the provided model, to determine a deviation between the desired angle of entrance and a current angle of entrance and to generate a signal depending on the determined deviation. This can ensure that the penetrating device is implanted into the tissue in an angle which allows to place the penetrating device along the provided path, thereby further increasing the accuracy of positioning the penetrating device within the tissue.

In an embodiment the tissue is bone and the system further comprises a proximity information providing unit configured to provide proximity information being indicative of a distance between the penetrating device and a cortical wall, wherein the proximity information has been determined based on a measurement carried out at a tip of the penetrating device, wherein the model providing unit is configured to provide the model such that it shows the cortical wall of the bone, wherein the processor is configured to determine a distance between the penetrating device and the cortical wall based on the provided model and the position of the penetrating device as indicated by the tracking information, to determine a deviation between the distance indicated by the provided proximity information and the determined distance and to determine an accuracy indicator being indicative of the accuracy of the generated virtual view based on the determined deviation. For instance, the proximity information can be determined based on impedance sensing. The accuracy indicator or a signal being dependent on the accuracy indicator like an optical and/or acoustical signal can be shown to the user. In particular, it can be indicated to the user if the accuracy indicator indicates an accuracy of the generated virtual view being smaller than a predefined threshold. This leads to a further improved assisting of the user while implanting the penetrating device. The processor can be configured to adapt the virtual view by adapting the pose of the penetrating device as indicated by the provided tracking information such that the accuracy indicator indicates an increased accuracy. This further increase the accuracy of the virtual view and hence further improves the assisting of the user while placing the penetrating device.

In an embodiment the system further comprises a tissue information providing unit configured to provide tissue type information about a tissue type as sensed by using the penetrating device, wherein the provided model shows different tissue types, wherein the processor is configured to determine an expected tissue type based on the provided model and the provided tracking information, to determine whether the expected tissue type and the tissue type defined by the provided tissue type information match each other and to, if the tissue types do not match, generate a signal indicating the mismatch. For instance, the signal can be an acoustical and/or optical signal for warning the user if there is such a mismatch indicating an inaccurate virtual view. In particular, a border of the virtual view can be colored with a first color if the tissue types match and with a second color if the tissue types do not match.

In an embodiment the system comprises a tissue information providing unit configured to provide tissue type information about tissue types as sensed by using the penetrating device, wherein the processor is configured to generate the virtual view such that it also indicates the sensed tissue types. Moreover, the model providing unit can be configured to provide the model such that it also shows a structure of risk, wherein the processor can be configured to determine for multiple regions of the model risk values depending on the distance of the respective region to the structure of risk and to indicate the determined risk values in the multiple regions in the virtual view. In an example the structure of risk can be the cortical bone, because the cortical bone should not be broken. However, the structure of risk could also be a blood vessel, the spinal cord, et cetera. The risk values can be indicated, for instance, by assigning the risk values to colors and by coloring the corresponding parts of the virtual view accordingly. This shows to the user which regions should be avoided while placing the penetrating device in the tissue, particularly in the vertebra, thereby providing a further improved assisting of the user in placing the penetrating device.

In another aspect of the present invention a method for assisting a user in placing a penetrating device in tissue is presented, wherein the method comprises:
- providing tracking information being indicative of a three-dimensional pose of the penetrating device,
- providing a three-dimensional model of the tissue,
- providing a path through the model,
- generating a virtual view from a perspective of a tip of the penetrating device within the tissue in the direction of the provided path based on the provided tracking information, the provided model and the provided path, wherein the virtual view is configured such that it indicates a direction in which the user should move the penetrating device while placing the penetrating device in the tissue.

In a further aspect of the present invention a computer program for assisting a user in placing a penetrating device in tissue is presented, wherein the computer program comprising program code means for causing a system as defined in any of claims 1 to 13 to carry out the steps of the method as defined in claim 14, when the computer program is run on a computer controlling the system.

It shall be understood that the system of claim 1, the method of claim 14 and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a system for assisting a user in placing a penetrating device in a vertebra,
Fig. 2 shows schematically and exemplarily a vertebra with a pedicle screw placed within the vertebra,
Fig. 3 shows an image of a vertebra with an inaccurately placed pedicle screw,
Fig. 4 illustrates a generation of a virtual view to be used for assisting a user in placing the pedicle screw in the vertebra,
Fig. 5 illustrates a determination of a path within the vertebra along which the pedicle screw should be arranged,
Fig. 6 illustrates a further determination of a path within the vertebra along which the pedicle screw should be arranged,
Fig. 7 illustrates how the generated view changes with forwarding the pedicle screw in a pedicle of the vertebra,
Fig. 8 illustrates a registration of an optical tracking device with pre-acquired medical image slices,
Fig. 9 illustrates a generation of a virtual view to be used for assisting a user in placing an instrument for generating a hole in a vertebra,
Fig. 10 schematically and exemplarily shows a pedicle screw with tissue sensing functionality,
Fig. 11 schematically and exemplarily shows the virtual view with differently colored borders,
Fig. 12 schematically and exemplarily shows a risk heat map overlaid on the virtual view, and
Fig. 13 shows a flowchart exemplarily illustrating an embodiment of a method for assisting a user in placing a penetrating device in a vertebra.

### DETAILED DESCRIPTION OF EMBODIMENTS

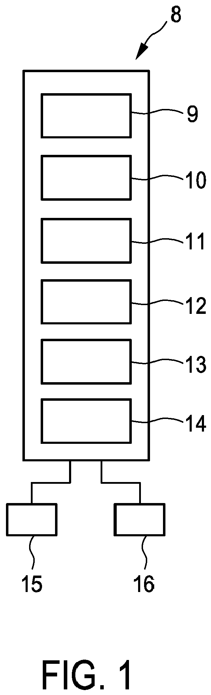

Fig. 1 shows an embodiment of a system as claimed for assisting a user in implanting a penetrating device in the form of a pedicle screw in a bone of a vertebra.

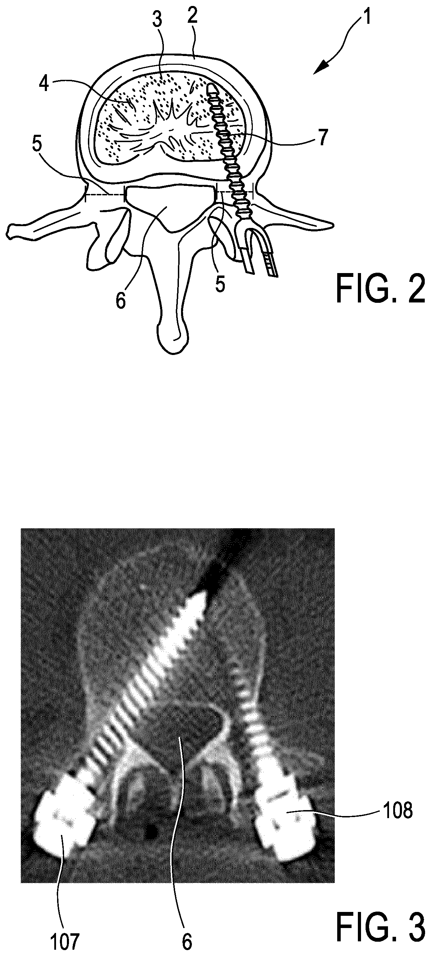

The vertebra 1 with a pedicle screw 7 are shown in Fig. 2. The vertebra 1 includes the cortical bone 2, the cancellous bone 3, the vertebral body 4, the pedicles 5 and the spinal cord 6. The pedicle screw 7 is typically placed in a slight medial angle passing through a pedicle 5 and into the vertebral body 4 up to the cortical bone 2 on the anterior side of the vertebra 1. In Fig. 2 the pedicle screw 7 is accurately positioned within the vertebra 1 when it penetrates the pedicle 5 in the middle region with some distance between the outer boundaries on the left and right hand sides as shown.

Fig. 3 shows a computed tomography image of a vertebra into which two pedicle screws have been implanted in an incorrect manner. The left pedicle screw 107 can be seen to pass through the left pedicle boundary and as a consequence partially penetrates the spinal canal 6 at its right side such that it has caused a medial breach before entering the vertebral body. This incorrect placement can cause nerve damage with many accompanying symptoms such as pain and loss of function. The pedicle screw 108 shown on the right has also been incorrectly placed. In this case the cortical bone at the screw's right side at the location of the pedicle 5 has been breached slightly. Although, this typically is a less severe medial breach than that by the screw 107, and often does not provide a reason to reperform surgery, it may still provide a less mechanically stable result compared to when the screw is accurately placed between both left and right extremities (outer walls) of the pedicle 5.

Embodiments of the system disclosed should assist a user like a surgeon in implanting a pedicle screw in a vertebra such that for example an incorrect placement as shown for screw 107 and/or 108 can be avoided or the extent of incorrect placement can be reduced.

The system 8 of Fig. 1 comprises a tracking information providing unit 9 configured to provide tracking information indicative of a three-dimensional pose of the penetrating device 7. In this embodiment the tracking information providing unit 9 is a receiver configured to receive tracking information from an optical tracking device 40 and to provide the received tracking information to the tracking information providing unit 9.

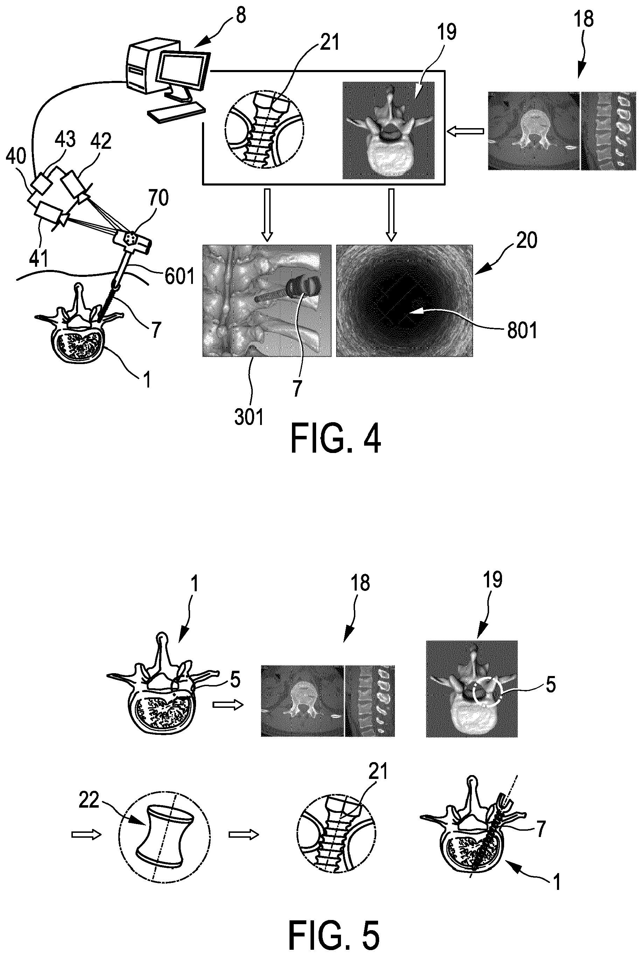

An embodiment of an optical tracking system 40 is shown in Fig. 4. It comprises two cameras 41, 42 and a processor 43 for determining the pose of a surgical instrument 601 used for implanting the pedicle screw 7 into the vertebra 1. The processor has one or more inputs 45 configured to receive the data of the cameras and an output for providing optical tracking data to

The surgical instrument 601 and/or the pedicle screw (when still in the field of view of the cameras) are visible in images acquired by the cameras 41, 42, and the processor 43 is configured to determine the pose of the surgical instrument 601 and hence therewith of the pedicle screw 7 and/or of the pedicle screw directly (when still in the field of view of the cameras) based on the images acquired by the cameras 41, 42. The processor may thus be capable of automatically recognizing the surgical instruments and their orientation within the images. Alternatively or additionally the instrument and/or pedicle screw may have markers 70 (for example optical markers) that may be recognized by the cameras and that are located on defined areas of the instrument and/or pedicle screw so that they may aid the determination of the orientation of the instrument and/or pedicle screw in the images. The processor 43 is further configured to transmit via its output the corresponding tracking information to the tracking information providing unit 9 of the system 8. As mentioned hereinbefore, the markers can be attached to the pedicle screw 7. However, it is preferred that the markers are provided on the instrument used to place the screw like a screw driver. The pedicle screw is typically rigidly attached to the instrument such that it is orientationally fixed with respect to the instrument. However, also other types of instruments, not being a screw driver, could be tracked for bone penetration.

In the embodiment a tracking device based on optical principles is used. However, alternatively or additionally tracking devices operating on different principles but capable of providing the pose can be used. For example, it is possible to use electromagnetic tracking devices, optical shape sensing tracking devices, Inertial movement unit based tracking devices et cetera.

In this embodiment, the pedicle screw 7 is rigidly connected to the surgical instrument 601, wherein the spatial relationship between the pedicle screw 7 and the surgical instrument 601 (including their mutual orientation) is known. The processor has an input for receiving such information e.g. provided by a user via user interface, or from a lookup table stored in a memory, or from determination of the images provided by cameras 41 and 42 when the screw and instrument are both in the field of view of the cameras. Using this spatial relationship data, the processor 43 can determine the pose of the pedicle screw 7 based on the pose of the instrument 601 as determined form the image data received from the cameras. The determined pose of the pedicle screw 7 can just refer to the location of the tip of the pedicle screw 7. However, it can also refer to the location and orientation of the pedicle screw 7, particularly of the entire pedicle screw 7.

The system 8 further comprises a model providing unit 10 which in this embodiment takes the form of a computer or data processor which is configured to provide a three-dimensional model 19 of the vertebra 1 from imagery obtained from the region comprising the vertebra of a subject in which a pedicle screw is to be implanted, wherein the model 19 shows the cortical bone 2 and possibly also the spinal cord 6. The imagery may be any type of imagery having characteristics suitable for generation of such model. It may for example be imagery obtained preoperatively from the relevant region of the subject using one or more of X-Ray, CT, MRI, Ultrasound, etc. modalities according to known principles. X-ray or CT imagery is often preferred because of higher resolution and/or contrast resulting possibly in a more precise model.

The computer or data processor is configured with an input to receive (in this case) X-ray imagery 18 of the vertebra 1 and is further adapted to segment the imagery 18 to generate the model 19 of the vertebra 1 based on the segmentation. For instance, the computer or data processor can be configured to process a set of anatomical 2D images within the imagery 18 for generating the model of the vertebra. Alternatively, the computer or data processor is configured to have an input to receive already segmented imagery so that the computer or data processor is adapted to generate the model based on the received segmented imagery. In all cases known segmentation techniques can be used for segmenting the different parts of the vertebra 1, and particularly for segmenting the cortical bone 2 and possibly also the spinal cord 6. The known segmentation techniques can be based on, for instance, shape-constrained deformable models as known in the art. Furthermore, the generation of three dimensional or two-dimensional representations of the model may be done using techniques as known in the art.

In an embodiment the model is a simple bone model of the vertebra, wherein the "void" in the vertebra that contains the spinal cord represents an area that the user needs to avoid penetrating during a procedure. In such case the spinal cord is not modelled or left out of the model such that it is not represented. The model can hence just be the segmented bone in the image which may simplify the segmentation and model generation, particularly in an X-ray based or CT based imagery.

The system 8 further comprises a path providing unit 11 configured to provide a path 21 through a pedicle 5 of the model 19 of the vertebra 1 where the path is a trajectory the pedicle screw should follow for an accurate placement in the vertebra. In particular, the path providing unit 11 is configured to calculate the path 21 based on the shape and dimensions of the pedicle as provided by the model 19. For example, the path providing unit 11 can be configured to map an hourglass-shaped model 22 to the pedicle 5 provided by the model and to calculate the path 21 based on the mapped hourglass-shaped model 22. This will be described in more detail with reference to Fig. 5.

Fig. 5 illustrates how a path 21, i.e. a desired pedicle screw trajectory, can be calculated. In the left upper corner of Fig. 5 the vertebra 1 with the pedicle 5 in which the pedicle screw 7 needs to be placed is illustrated. From X-ray or CT imagery 18 of the vertebra region acquired of a subject, a three-dimensional model 19 of the vertebra 1 is generated in which the pedicle 5 is modeled in three dimensions. An hourglass-shaped model 22 of the pedicle 5 is fitted to the pedicle 5 using volumetric or 3D registration. Thus, the shape of the pedicle 5 is mapped to an hourglass-shaped pedicle model 22 or vice versa by using the volumetric registration. The path 21 is then determined such that it follows the rotational axis of the hourglass-shaped pedicle model 22 through the center of the hourglass-shaped pedicle model 22. If this path, i.e. the desired trajectory, is followed by the user, the pedicle screw 7 is placed straight through the center of the pedicle 5 with a minimum risk of breaching the cortical bone. In addition, the hourglass-shaped pedicle model may be oriented such that the desired trajectory to be followed by the pedicle screw in combination with the length of the pedicle screw in relation to the dimensions of the vertebra is chosen such that when inserted, the pedicle screw front tip just reaches the cortical bone 2 at the side of the vertebra opposite to the entrance location. This provides good stability of the screw within the vertebra.

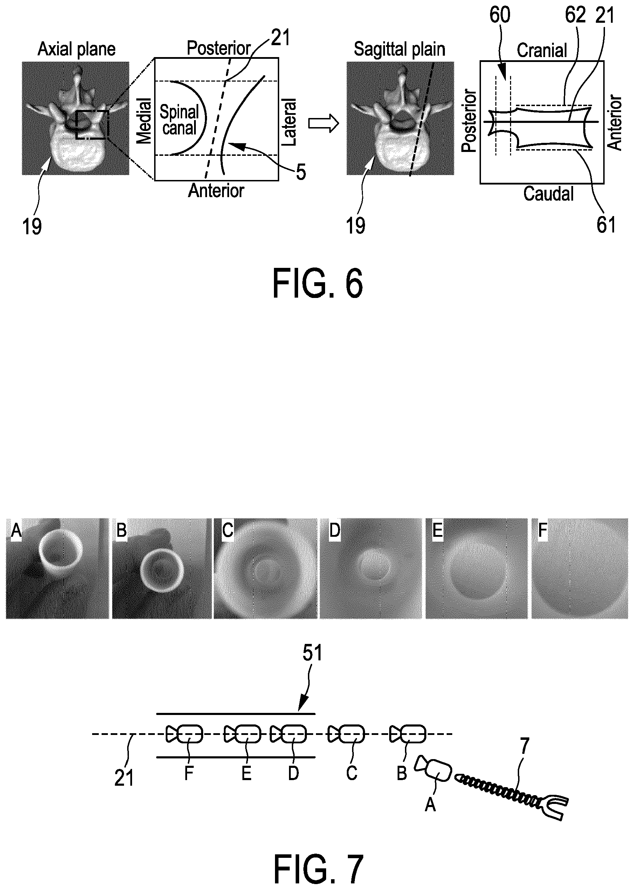

The path providing unit 11 can also be adapted to determine the path in another way. For instance, the technique disclosed in WO 2017/186799 A1, which is herewith incorporated by reference, could be used for determining the path. The path providing unit 11 can also be configured to calculate the path 21 further based on the orientation of end plates of the body of the vertebra as provided by the model 19. The vertebral end plates, i.e. the end plates of the body of the vertebra, are the top and bottom portions of the vertebral bodies that interface with the vertebral discs, if a person is standing. The path providing unit 11 can be adapted to firstly determine an ideal direction of the pedicle screw 7 based on the hourglass-shaped pedicle model 22 as described above in an axial plane. This is schematically and exemplarily illustrated in the left part of Fig. 6. The path providing unit 11 can be further configured to secondly align the direction of the screw path 21 parallel to the end plates 61, 62 of the vertebral body as obtained from the vertebral segmentation, i.e. as provided by the model 19. This aligning is illustrated in the right part of Fig. 6 in which reference sign 60 indicates the spinal canal. The end plates 61, 62 can be defined as two parallel planes arranged on opposite sides of the respective vertebra, wherein these sides of the vertebra are the sides where the intervertebral discs are located.

### Perspective view providing unit

The system 8 further comprises a processor 14 configured to generate a virtual view 20 from a perspective of a tip of the pedicle screw 7 where the virtual view includes at least partially is in the direction of the path 21. The virtual view based on the provided tracking information, the provided model 19 and the provided path 21. For generating the virtual view the tracking information, the provided model and the provided path are registered to each other. If the provided model and the provided path both have been generated based on a same image of the vertebra or if the provided path has been generated based on the provided model, the provided model and the provided path are automatically registered to each other. Otherwise, they could be registered to each other by using known registration techniques. For registering the tracking information with the provided image and hence with the provided model and the provided path the tracking device 40 can be registered with the image, for instance, by using markers which are identifiable in the image and trackable by the tracing device 40. However, also other known registration techniques are possible.

The registration between, for instance, image data coordinates of the provided image 18 and coordinates of the pedicle screw 7, particularly of the tip of the pedicle screw 7, obtained with the tracking device 40 provides a corresponding coordinate mapping which can be used by the processor 14 for generating the virtual view 20, i.e. to provide the user with visual navigation guidance. In other words, the coordinate mapping between the provided image 18 and hence between the three-dimensional vertebra model 19 and the path 21 and the tip coordinates of the pedicle screw 7 provided by the tracking device 40 can be used to provide the user with the virtual view 20 from the perspective of the tip of the pedicle screw 7.

The virtual view 20 is generated such that it indicates a direction in which the user should move the penetrating device while placing the penetrating device in the vertebra 1. In particular, the processor 14 is configured to generate the virtual view 20 such that it shows a virtual tunnel 801 within the vertebra 1, wherein the virtual tunnel 801 is arranged along the provided path 21 such that the virtual view 20 is a view in the virtual tunnel 801 in the direction of the provided path 21, in order to indicate a direction in which the user should move the penetrating device while placing the penetrating device in the vertebra 1. It should be noted that the virtual tunnel 801 is not really present. It is just used for intuitively guiding the user. In another embodiment the virtual view could also be generated such that it indicates a direction in which the user should move the penetrating device while placing the penetrating device in the vertebra in another way. For instance, generally the virtual view can show an element which the user should center, i.e. the user should maneuver the penetrating device such that the element is located centrally in the generated virtual view 21. This element to be centered could also be a virtual end of the virtual tunnel.

The virtual view 20 corresponds to a virtual view point inside the vertebra 1 such that a screw tip perspective or an internal perspective near the screw tip is generated. This representation from an internal perspective is different compared to two-dimensional cross sections or three-dimensional contour images which might generally also be used in navigation-assisted spine surgery procedures. In this embodiment the pedicle 5 is presented in the virtual view 20 as a tube or tunnel, wherein preferentially the inner part of the tunnel has a different appearance like a different color than the distal part of the tunnel. This will in the following further be explained with reference to Fig. 7.

Fig. 7 schematically and exemplarily illustrates what a virtual view (pictures A to F at the top of the Fig. 7) would look like from the tip of a pedicle screw in the direction of the longitudinal axis of the pedicle screw by means of use of a camera (representing the virtual view) during the several stages of navigating the pedicle screw (camera) towards and through the pedicle (the tube representing the outer boundaries of the pedicle and being aligned along the calculated path the pedicle screw needs to follow for correct implantation. Thus, the virtual views A to F at the top correspond with the situations A to F at the bottom part of Fig. 7. Thus, the virtual view can be used as a guide for the navigation of the screw towards the desired pedicle entry point and for its orientation and line up with the desired path such that it penetrates along the desired path. In particular, while approaching the pedicle from a posterior side (pose A), the virtual view assists in aligning the instrument with the planed trajectory (pose B). By centering the "end of the tunnel" (poses C to F) the user will automatically follow the planned trajectory, i.e. the planned path 21.

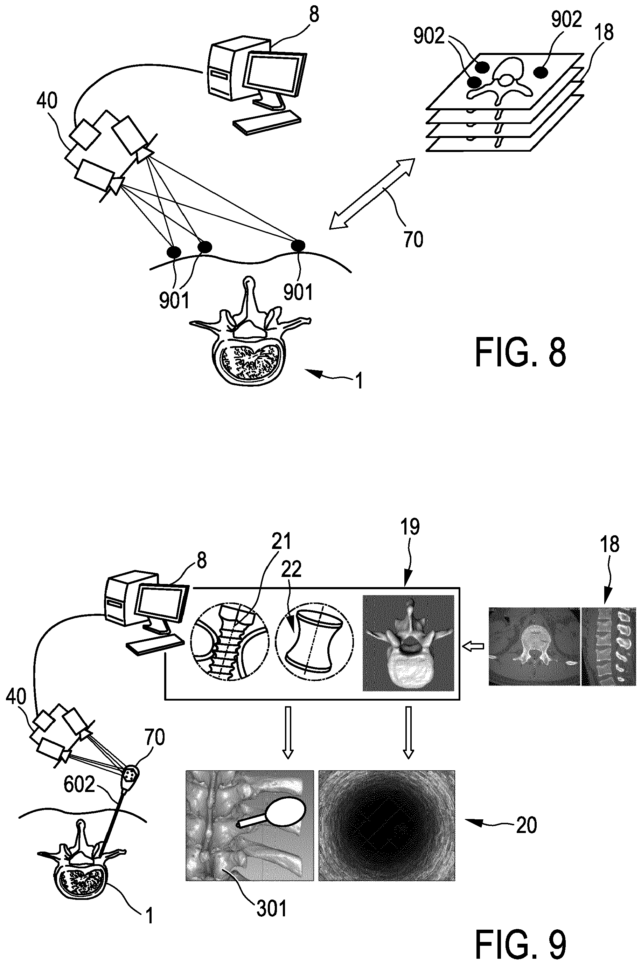

As explained hereinbefore, the tracking information, the generated model and the calculated path are registered to each other. This registration may be done in any way suitable. In one preferred embodiment use is made of a registration based on a mapping between a coordinate system of the optical tracking device 40 and a coordinate system of the imagery 18 aided by fiducials or markers that are visible or identifiable in the imagery 18 as well as in the tracking data provided by the tracking device. An example of this preferred mapping will be described with reference to Fig. 8.

In Fig. 8 it is illustrated that the optical tracking system 40 tracks at least the position and optionally, but preferably possibly also the orientation of fiducials 901 on a patient's body, wherein these fiducials 901 are also visible in the imagery 18 as fiducial images 902. The Imagery 18 in this case is a three-dimensional image set having a stack of two-dimensional images, for example recorded using one or more of the aforementioned imaging modalities. As indicated before, the tracking device 40 also record the fiducial 901 locations and optionally, but preferably, orientations to incorporate these in the tracking information. Thus, the positions and, if also tracked, the orientations of the fiducials 901 are known in the coordinate systems of the optical tracking system 40 and of the imagery 18 and can be used as mutual references by the processor 14 of the system 8 for registering (segments of) the tracking information or segments thereof with the imagery or segments thereof. In Fig. 8 the registration is indicated by the double arrow 70.

In the embodiment described with reference to, for instance, Fig. 4 the penetrating device is a pedicle screw. However, in general with the invention or its embodiments, the penetrating device can also be another device, such as for example an instrument that is adapted to create a hole in a pedicle. Examples of such instruments are drills, awls, pedicle probes, k wires et cetera. As schematically and exemplarily shown in Fig. 9, the instrument 602 for pedicle hole creation comprises optical markers 70 to be tracked by the optical tracking device 40, wherein the optical markers 70 are rigidly connected to the proximal part of the instrument 602, i.e. the part opposite to the distal part also called the tip for protruding into the vertebra. The tracking technique of this instrument 602 may be done in a similar way as described for the instrument 601 and/or pedicle screw 7 in Fig. 4. Thus, the virtual view guidance of navigation of the instrument 602 during treatment may be performed in the same manner as described herein before for guidance of the navigation of the instrument 601 and/or the pedicle screw.

In case a treatment requires multiple manipulations such as hole creation and screw implantation, the registration for both may require different tracking data as he instruments may differ from each other and the procedures happen time sequentially, but may make use of the same imagery 18 and may make use of the same created model and/or calculated path. Note however that in some cases the boundaries of a projected tube in a view may differ.

The virtual view herein above included a tunnel representation of the calculated path and boundaries. Other indicators may be additionally or alternatively present. Thus, indicators depicting the planned path and/or a target region. Also the direction of the virtual view perspective may be indicated with a point in the middle or a perspective axis which is aligned with the instrument's penetration direction such as hole creation direction or pedicle screw axis. Such indicators can be calculated by a processor and provided as overlays to the virtual view as output to the user.

The processor can optionally be configured to generate a further virtual view for representing an external view, i.e. a view from an external view point different form the tip of the screw view point. An example of such further virtual view 301 is illustrated in Figs. 4 and 9. In this view a model of a part of the relevant anatomy is calculated form the same imagery 18 and tracking information as used for calculating the model 19, albeit that different parts (e.g. different segmentations) of such imagery and information may be used when the models differ. If the models are the same, the same model may be used to generate a different perspective and virtual view with. Again, the calculated path and a virtual axis along the instruments penetration path may be shown as overlays in good registration in the further virtual view.

In an embodiment, both the further virtual view 301 and the virtual view 801 are provided to the user.

In all described embodiments and others, the calculation of path and generation of virtual views may be done more than once. Preferably they are done periodically or even continuously such as in real time. The more updates or regenerations per time the better the guidance of navigation may be performed. In this way a movie style representation of the guidance during the actual treatments performed by a user such as medical practitioner can be provided. Preferably

The system 8 also comprises one or more user interface 15 for input of data by a user such as for example a mouse, keyboard, voice control, touchpad etc. and/or one or more user interfaces XXX for output of data to a user such as for example a speaker, tactile output device or one or more displays for showing still or video form of the virtual views.

The system 8 can further comprise a tissue information providing unit 13 configured to provide tissue type information being indicative of a tissue type as sensed by a penetrating device during use. In particular, the tissue information providing unit 13 is adapted to receive tissue information from a processor 102 which has determined the tissue type based on spectral characteristics of received light. Thus, in this embodiment the tissue information providing unit 13 is a receiver for receiving corresponding information from the processor 102 which is adapted to process signals received from the tip of the penetrating device for determining the tissue type information.

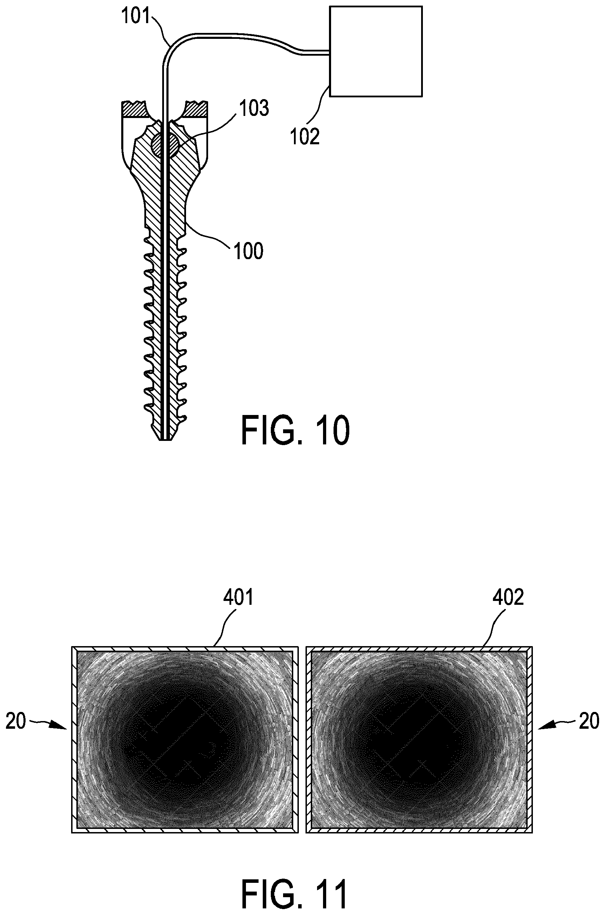

Such a processor 102 is schematically and exemplarily shown in Fig. 10.

An example of a tissue information providing unit is shown in Fig. 10. In this case the unit is shown in combination with a pedicle screw 100, but it can be used with other instruments. The pedicle screw has a lumen or hollow shaft 103 within it that extends along the longitudinal central (central positioning is not necessarily) axis of the pedicle screw and has openings at the distal (tip) end of the screw and the proximal (head) end of the screw. Within this shaft is positioned a stylet with a tip 104 of an optical sensor device 101 of the unit 13. The stylet is positioned such that the optical sensor is capable of sensing optical signals at the its tip 104 from the external of the distal screw tip and, optionally, is also capable of emitting optical signals from this stylet tip into the external of the distal screw tip. The optical device further includes a wave guide (e.g. optical guide) that is removably connected to an input/output of a signal receive/send interface 102. The waveguide is adapted to transport (e.g guide) the optical signals from the interface 102 to the tip of stylet and any sensed optical signals from the tip of the stylet to the interface 102. In this case the optical device 101 is removable from the shaft 103 of the pedicle screw, such that for example after placement of the pedicle screw in a pedicle it may be removed from the pedicle screw and possibly be used again for placement of a next pedicle screw. A sliding action may be used for removal and/or insertion of the optical device at the proximal opening of the shaft. There may be locking mechanism of any suitable kind (e.g. clamp located at the screw head) used to temporarily fix the optical device (or the stylet of this optical device) within the shaft, thus facilitating use of the unit 13 during placement of the screw by the user.

The signal receive/send interface is adapted to receive from and, optionally but preferably, also send optical signals into the waveguide of the optical sensor device. The interface is also capable of outputting a sensed data signal based on the received optical signals which sensor data signal that can be signal processed by a processor. Likewise, if the interface is also capable of sending optical signals it is adapted to generate such optical signals from received send data signals that can be processed by a processor.

The signal data processor is adapted to process the sensed data signal to extract from it one or more parameters indicative of the tissue type in front of the tip of the pedicle screw 207. Refer to later explanation or provide an embodiment here. However, the interface and therewith the unit 13 can also include the signal data processer. In

The one or more indications are subsequently transmitted to a further part of the system 8.

Typically, the interface 102 may thus be as simple as capable of converting optical signals into electrical signals (for processing) and vice versa and thus operate as a signal converter. The signal data processor may in such case be part of and even integrated in one central processor of the system. In another embodiment, the signal data processor may be part of the interface 102 and/or the unit 13. The unit 13 then provides the indications of the type of tissue to the central processor.

It will be clear that other ways of implementing a tissue sensing functionality may also be employed. Thus for example a diode type sensor may be used which does not necessarily have a waveguide and which has its interface located at the screw tip while electrical leads are led through the shaft to connect to the processor 102. Yet other sensory embodiments may be used.

The penetrating device with the sensing functionality as exemplified above can be used together with the virtual view generation principles explained with respect to Figs. 4 and 9. To this end, the sensed tissue type information is registered with the virtual view by making use of the tracking information. For example by logging time stamps of optical measurements (acquisition of optical sensor data) and compare such timestamps with those of a particular tracking information enables deduction of a pose during which the measurement was done. Hence a tissue type sensed at one particular instance may be correlated with a pose of that particular instance just as the geometry of penetrations may be correlated with pose as described herein before.

In an embodiment, the model 19 is provided such that it shows so called expected tissue types of the vertebra 1 for different locations within the vertebra 1. Such expected tissue types may be based on predetermined data known from external databases or otherwise available pre-operative data. For example some types of imaging modalities may provide imagery 18 that includes spatially resolved tissue type data. Particular examples may be spectral CT or MRI. In this embodiment, the processor 14 is adapted to determine one or more expected tissue types that should appear at certain locations within a virtual view that belongs to a particular pose, for example it may determine expected tissue types at the location within the view that corresponds with the calculated path and/or the outer boundaries of the tunnel view. The system is then also configured to be capable of comparing the expected tissue types with the ones determined using the optical sensor device and of providing one or more match indications to a user indicative of a degree of match of the expected and sensed tissue types that correspond to a particular location within the virtual view and/or on the model shown. If there is no or a pour match, the match indication may represent this with a corresponding output to a user and likewise if the match is good or perfect another match indication may be provided. The output may be binary as to indicate bad match/good match or with multiple levels or continuous levels to indicate further degree of matching. In such case any indication that can represent such scale of match may be used such as for example digital or analog output of number or a color scheme such as with heat maps. Other outputs may be used also. For example an output may be used number or analog color scheme or number scheme The user output may be in the form of, such as for example a tactile output, an audio output, or a visual output. Especially the visual output is preferred. It can be provided as part of the virtual view.

One preferred embodiment for proving a match indication output to a user is described with Fig. 11. Fig. 11 shows two virtual views 20. In the left shown view the measured and expected tissue types have a match that is considered good and this is indicated by the border of the virtual view 20 having a first color 401. In the left shown view the measured and expected tissue type have a match that is considered less good than the one of the left shown view and therefore the border of the right shown virtual view 20 is colored with a second color 402 different from the first color. As indicated herein before, a degree of matching can be represented using a heat map coloring scale, where for example red indicates less good match (as with 402) than green or blue (as with 401).

Thus, coloring the border of the virtual view can be used to indicate whether a tissue sensing signal at the tip of the penetrating device is congruent with information initially obtained from medical imaging. For example, one color like green 401 could mean that the bone type at the tip as derived from the model and hence from medical imaging and navigational information matches with the bone type that is estimated based on tissue sensing at the tip. In the same way, a mismatch at the two types of information results in another color like the color red 402 to warn the user about this inconsistency. Of course different types of shading can alternatively or additionally be used in the above embodiment.

The tissue sensing can be based on, for instance, diffuse reflectance spectroscopy (DRS), ultrasound, impedance measurements, et cetera. References

The processor 14 can be further configured to generate the virtual view 20 such that it also indicates one or several sensed tissue types. In particular, the tissue type information, i.e. the tissue sensing information, can be co-registered with the tracking information, i.e. with three-dimensional spatial information obtained by the optical tracking device 40, allowing to map the tissue sensing information onto a three-dimensional coordinate system and to create a tissue-sensing enriched virtual view of the scene.

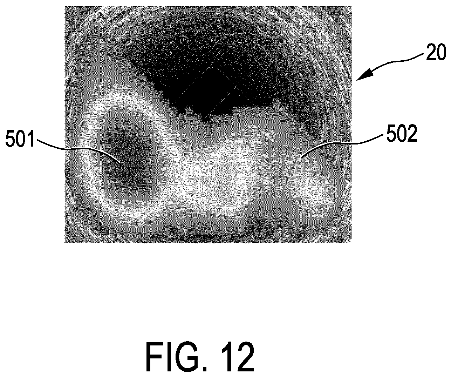

The processor 14 can be further configured to determine for multiple regions of the vertebra risks of creating a breach if touched by the penetrating device based on the provided tissue type information and the provided tracking information and to indicate the determined risks in multiple regions in the virtual view. Thus, the tissue type information, i.e. the tissue sensing information, can be used to calculate a risk of creating a breach in the cortical bone. The three-dimensionally mapped tissue sensing information and derived parameters like the breach risk can be translated to color tones for generating, for instance, a heat map that is projected and/or overlaid on the virtual view presented to the user. For example, as schematically and exemplarily illustrated in Fig. 12, regions having a higher risk of breaching can be colored in a specific color 501 like red, whereas regions with lesser risk of creating a cortical breach can be colored in another color 502 like green.

Thus, at the tip of the bone penetrating device there can be a tissue sensor that provides a measure for the risk of breaching. For instance, by using optical spectroscopy or electrical impedance the tissue type can be estimated, in order to indicate whether the tip is close to cortical bone or not. Still, this is a one-dimensional, i.e. point, measurement. Before penetrating the tissue, no sensor data is available, so the three-dimensional model is not yet enriched with this information. If the pose, i.e. the three-dimensional position and orientation, of the instrument tip is recorded over time during the procedure, this can be used to fill a three-dimensional coordinate system of the model with data points that represent the risk of breaching. The risk of breaching can be calculated, for instance, based on the distance to cortical bone, i.e. the risk can increase with decreasing distance to cortical bone. In this way, during the procedure sensor data can be used to provide the three-dimensional model with colors, wherein a respective color represents a respective risk. This coloring can be done in real-time and shown to the user as indicated in Fig. 12 while penetrating the tissue and the colors can preferably remain there until the end of the procedure; unless there is a good reason to "forget" certain information over time; e.g. in case there is reason to believe that over time data is becoming less reliable; or new data at the same pose that can be used to update the risk profile.

It should be noted that typically the tip of the penetrating device cannot be moved freely around in bone, as it is not a "void". However, it could happen that a device while being placed has come too close to the wall of the virtual tunnel and the user has decided to retract the penetrating device and re-insert it in a different way. In that case the method of coloring certain parts of the wall/tunnel that have been touched and found to be risky is very helpful.

The model providing unit 10 can be configured to provide the model such that it also shows critical structures like blood vessels in the surrounding of the vertebra. The processor 14 can be configured to also show the critical structures in the generated virtual view. Thus, the virtual view from the device-tip perspective can be enriched by further anatomical information obtained from the medical image 18. For instance, based on the three-dimensional medical image data 18 not only the bone of the vertebra 1 of interest is segmented, but also further critical anatomical structures like the aorta, the spinal canal, et cetera. For instance, the processor 14 can be configured to render the pedicle wall semitransparent and depict the critical structures behind the wall. The processor 14 can also be configured to translate the spatial relation between the pedicle wall and the critical structures into a three-dimensional risk map which can be translated to a color scheme for generating a heat map which can be projected and/or overlaid on the virtual view that is presented to the user. Also further information like bone quality parameters, for instance, osteoporosis, density, et cetera, which can be obtained from the medical image 18, can be used to enrich the virtual view that is presented to the user. The processor 14 can be configured to color regions that are close to vital anatomical structures with a specific color like red and regions which are not close to vital anatomical structures with another color like green. Thus, the processor 14 can be configured to calculate risks depending on distances of shown regions to vital anatomical structures and to color these regions depending on the determined risks.

In an embodiment the system 8 can also comprise a proximity information providing unit 12 configured to provide proximity information being indicative of a distance between the penetrating device, particularly the tip of the penetrating device, and the cortical wall, wherein the proximity information has been determined based on a measurement carried out at the tip of the penetrating device. For instance, the penetrating device can be adapted to sense an electrical impedance at the tip of the penetrating device, wherein the penetrating device can be electrically connected to a processor which provides the electrical current for measuring the electrical impedance and which processes the measured electrical impedance for determining the distance between the penetrating device and the pedicle cortical wall. This distance can be transmitted to the proximity information providing unit 12 as the proximity information, which can then provide the proximity information.

The processor 14 is configured to determine a further distance between the penetrating device and the pedicle cortical wall based on the provided model 19 and the position of the penetrating device as indicated by the provided tracking information. The processor 14 can then be adapted to determine a deviation between the distance indicated by the provided proximity information and the determined further distance and to determine an accuracy indicator being indicative of the accuracy of the generated virtual view 20 based on the determined deviation.

Thus, the penetrating device like the pedicle screw or the instrument for creating a hole as described above can be equipped with tissue sensing, for instance, based on spectral tissue sensing or impedance sensing allowing for a direct distance measurement of, for instance, the tip of the penetrating device with respect to the pedicle cortical wall. In case of the impedance sensing or another sensing carried out by the penetrating device which allows for a distance determination, the signal deduced from this sensing can be compared with the distance determined by the tracking device, wherein the amount of difference between the two measurement systems, i.e. tracking device and directly measuring the distance via, for instance, impedance sensing, is an indication of the accuracy of the provided device-tip perspective view 20. This accuracy can be indicated in the view by giving the view or part of the view a distinct feature, for example, by coloring the edge of the virtual view 20 depending on the determined deviation.

The processor 14 can be further configured to adapt the virtual view 20 by adapting the pose of the penetrating device as indicated by the provided tracking information such that the accuracy indicator indicates an increased accuracy. In particular, if the determined distance is larger than a predefined threshold, it can be assumed that the tracking information and/or the registration is not accurate enough. In this case the assumed pose of the penetrating device within the vertebra, which is used for generating the virtual view 20, can be modified such that the deviation becomes below the predefined threshold and particularly is zero. This modified assumed pose of the penetrating device can then be used for generating the virtual view 20.

Thus, the system can use, for instance, tissue sensing or distance sensing for assessing the quality of the pose estimation of the penetrating device and use the result of this assessment for determining how to adjust the pose estimation, i.e. the assumed current pose such that it would better match the tissue or distance sensing information.

The processor 14 can be further configured to generate a visualization indicating a geometrical relationship between a current pose of the penetrating device and a target pose of the penetrating device as defined by the path based on the tracking information and based on the provided path. The processor 14 can also be adapted to generate a visualization indicating a geometrical relationship between the current pose of the penetrating device and provided poses of regions of interest within the vertebra based on the tracking information and based on the provided poses of the regions of interest. The generated visualization can include an overlay of the geometrical relationship over the virtual view. For instance, the geometrical relationship can be indicated by using a bulls-eye view or target point, which is overlaid on the virtual tunnel.

The processor 14 can also be configured to determine a distance between a central axis of the pedicle and the position of the penetrating device as indicated by the tracking information based on the provided model and the provided tracking information and to generate a signal based on the determined distance. For instance, the processor 14 can be adapted to provide an optical and/or acoustical signal if this distance is larger than a predefined threshold, in order to indicate that the distance to the central axis of the pedicle is too large.

Moreover, the processor 14 can be configured to determine a desired angle of entrance of the penetrating device into the vertebra based on the provided path and the provided model, to determine a current angle of entrance of the penetrating device into the vertebra based on the provided tracking information and the provided model and to determine a deviation between the desired angle of entrance and a current angle of entrance and to generate a signal depending on the determined deviation. For instance, also here an optical and/or acoustical signal can be provided, in order to warn the user, if this deviation is larger than a predefined threshold.

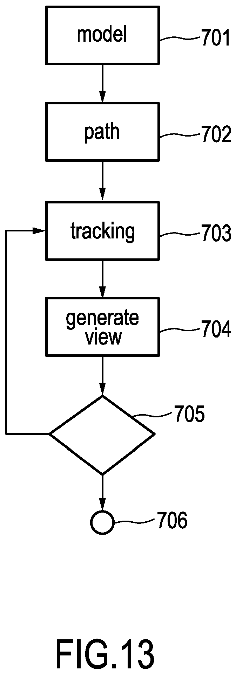

An embodiment of a method for assisting and/or guiding a user in placing a penetrating device in an anatomical part/volume of a subject such as for example a vertebra or other skeletal part of a subject will be described with reference to the flow chart of Fig. 13.

In step 701 a three-dimensional model of the anatomical part is generated or provided. In line with such generation described herein before, such model may be generated using pre-operatively obtained imagery 18 of the anatomical part obtained using the relevant imaging modalilty, wherein the model is configured to show at least the bone part of the vertebra. Preferentially the model also shows further parts like the cancellous bone, the spinal cord or other critical structures like surrounding blood vessels.

In step 702 a path through a pedicle of the model is provided, wherein the path is preferentially calculated such that it follows the central axis of the pedicle. In step 703 tracking information is provided, which is indicative of the three-dimensional pose of the penetrating device.

In step 704 a virtual view from a perspective of a tip of the penetrating device within the vertebra in the direction of the provided path is generated based on the provided tracking information, the provided model and the provided path.

In step 705 it is determined whether an abort criterion has been fulfilled, wherein, if the abort criterion has not been fulfilled, the method continues with step 703. Thus, steps 703, 704 and 705 are carried out in a loop such that a user can be assisted by looking at the generated view shown on a display while placing the penetrating device. If the abort criterion is fulfilled, the method stops in step 706. The abort criterion can be, for instance, whether a user has indicated via the input unit that the method should be stopped.

Generally, in thoracic spine pedicle screw placement, accuracy is a challenge because of the morphology, particularly of the relatively small pedicle size. Lateral or medial cortical breaches in pedicles, particularly in thoracic pedicles, can potentially yield severe clinical complications due to the proximity of critical structures. It is a challenge for a user to navigate the penetrating device through a narrow pedicle without creating a breach. Furthermore, the user has to reach as close as possible to the cortical bone on the anterior side of the vertebral body as indicated in Fig. 2. As generally the user is unable to observe the progress directly, these two tasks are very difficult in practice. There is therefore a need for a system to spatially guide the user during such a procedure. This need is fulfilled by the system for assisting a user in placing a penetrating device in a vertebra as described above with reference to, for instance, Figs. 1, 4 and 9.

The system for assisting a user in placing a penetrating device uses a tracking of the pose of the penetrating device and a three-dimensional image of the vertebra, wherein a segmentation algorithm is used to segment the cortical bone of the vertebra and preferentially also the spinal canal and major blood vessels, in order to generate a three-dimensional model of the vertebra. An algorithm is used to calculate the path through the pedicle as shown by the three-dimensional model, wherein the penetrating device is related relative to the segmented vertebra, i.e. relative to the model. Based on the segmentation of the vertebra, i.e. based on the three-dimensional model, and the three-dimensional pose as provided by the tracking system a virtual view is created from the perspective of the tip of the penetrating device, wherein the virtual view indicates a path through the pedicle and preferentially critical anatomical structures along the way.

This representation from an internal perspective is different compared to, for instance, two-dimensional cross sections, three-dimensional contour images or camera image overlay methods. As mentioned above, the virtual view can include a visible indication of the geometrical relationship between the current pose of the penetrating device and either a pre-planned path to a desired tip pose or one or more locations of interest indicated in the provided, i.e. pre-acquired, medical image which might be a computed tomography image or a magnetic resonance image. This might be provided to the user in the form of a graphic overlay over the simulated internal view from the perspective of the tip of the penetrating device, or as a highlighted location on the simulated external view, or both. A signal, which might be a color signal or a sound signal, can be provided to the operator when the penetrating device is close to the central axis of the pedicle. Moreover, a signal can be provided when the angle of entrance is optimal. The rendering of the internal view could be done based on photo-realistic rendering.

The penetrating device can be, as also mentioned above, equipped with tissue sensing, for instance, with spectral tissue sensing or impedance sensing, wherein the impedance sensing could also be used to sense the proximity to the pedicle cortical wall. The tissue sensing can also be used for assessing the quality of the pose estimation, i.e. of the determination of the pose of the penetrating device defined by the tracking information, wherein the result of this assessment can be used for determining how to adjust the pose estimation such that it better matches the tissue sensing information.

Although in above described embodiments the bone is the bone of the vertebra, in other embodiments the bone can also be the bone of another part of a person, in order to assist the user in placing the penetrating device in the bone of this other part of the person. Moreover, instead of placing the tissue-penetrating device in bone, i.e. in bony tissue, it can also be placed in another type of tissue.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Determinations like the determination of the path, the determination of the model, the determination of the tip-perspective view, et cetera performed by one or several units or devices can be performed by any other number of units or devices. These determinations and/or the control of the system for assisting a user in placing a penetrating device in a bone in accordance with the method for assisting a user in placing a penetrating device in a bone can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for assisting a user in placing a penetrating device in tissue like a pedicle screw in a vertebra's pedicle. The system generates a virtual view from a penetrating device tip perspective within the tissue in the direction of a path through a model of the tissue. The virtual view is generated based on tracking information indicating a pose of the penetrating device, the model and the path, wherein the virtual view is configured such that it indicates a direction in which the user should move the penetrating device while placing it in the tissue. For instance, it can show a virtual tunnel which is arranged along the path. If a user like a surgeon is provided with such a virtual view, the user can position the penetrating device along the path with a significantly increased accuracy.

We need definitions of processor, memory and input/output/ user interface. There may be wired and wireless connections in a direct way or via data network.

A processor 222 or a processor 292 for a controller is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A processor is an article of manufacture and/or a machine component. A processor 222 for a controller 220 is configured to execute software instructions to perform functions as described in the various embodiments herein. A processor 22 for a controller 220 may be a general-purpose processor or may be part of an application specific integrated circuit (ASIC). A processor 222 for a controller may also be a microprocessor, a microcomputer, a processor chip, a controller, a microcontroller, a digital signal processor (DSP), a state machine, or a programmable logic device. A processor 222 for a controller may also be a logical circuit, including a programmable gate array (PGA) such as a field programmable gate array (FPGA), or another type of circuit that includes discrete gate and/or transistor logic. A processor 222 for a controller may be a central processing unit (CPU), a graphics processing unit (GPU), or both. Additionally, any processor described herein may include multiple processors, parallel processors, or both. Multiple processors may be included in, or coupled to, a single device or multiple devices. A "processor" as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each including a processor or processors. Many programs have instructions performed by multiple processors that may be within the same computing device or which may even be distributed across multiple computing devices.

Memories such as the memory 221 or the memory 291 described herein are tangible storage mediums that can store data and executable instructions and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A memory described herein is an article of manufacture and/or machine component. Memories described herein are computer-readable mediums from which data and executable instructions can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, Bluray disk, or any other form of storage medium known in the art. Memories may be volatile or non-volatile, secure and/or encrypted, unsecure and/or unencrypted. "Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

## Claims

1. System for assisting a user in placing a penetrating device (7) in tissue of a subject, the system (8) comprising:
- a first input for receiving tracking information being indicative of a three-dimensional pose of the penetrating device (7),
- a second input for receiving a three-dimensional model (19) of the tissue,
- a third input for receiving a path (21) through the model (19),
- a processor (14) configured to generate a virtual view (20) comprising a representation of the provided path (21) from a perspective of a tip of the penetrating device (7) based on the received tracking information, the received model (19) and the received path (21), wherein the virtual view is configured such that it indicates a direction in which the user should move the penetrating device while placing the penetrating device in the tissue, and optionally
- a user interface for providing the virtual view to a user such as medical practitioner or subject.

2. The system as defined by claim 1 or 2, wherein the processor (14) is configured to generate the virtual view (20) such that it shows a virtual tunnel (801) within the tissue, wherein the virtual tunnel (801) is arranged along the received path (21) such that the virtual view (20) is a view in the virtual tunnel (801) in the direction of the received path (21), in order to indicate the direction in which the user should move the penetrating device while placing the penetrating device in the tissue.

3. The system as defined by claim 2, wherein the tissue is bone and the model provider (10) is configured to provide the model such that it distinguishes between a first bone tissue type having a first density and a second bone tissue type having a second density, wherein the second bone tissue type at least partly encloses the first bone tissue type, wherein processor (14) is configured to generate the virtual tunnel (801) such that an inner hollow part of the tunnel (801) represents the first bone tissue type and an outer wall of the tunnel (801) represents the second bone tissue type.

4. The system as defined by any of the preceding claims, wherein the tissue is bone of a vertebra comprising a pedicle, wherein the model providing unit (10) is configured to provide a three-dimensional model (19) of the bone of the vertebra (1) comprising the pedicle as the model and wherein the path providing unit (11) is configured to calculate the path (21) based on the shape and dimensions of the pedicle as provided by the model (19).

5. The system as defined by claim 4, wherein the path providing unit (11) is configured to map a hourglass-shaped model (22) to the pedicle provided by the model and to calculate the path (21) based on the mapped hourglass-shaped model (22).

6. The system as defined by any of claims 4 and 5, wherein the path providing unit (11) is configured to calculate the path (21) further based on the orientation of end plates of the body of the bone of the vertebra as provided by the model (19), wherein the end plates are on top of and below the vertebra, when a person is standing.

7. The system as defined by any of the preceding claims, wherein the processor (14) is configured to determine a distance between the provided path and the position of the penetrating device (7) as indicated by the tracking information based on the provided model (19) and the provided tracking information and to generate a signal based on the determined distance.

8. The system as defined by any of the preceding claims, wherein the processor (14) is configured to determine a desired angle of entrance of the penetrating device (7) into the tissue based on the provided path (21) and the provided model (19), to determine a current angle of entrance of the penetrating device (7) into the tissue based on the provided tracking information and the provided model (19), to determine a deviation between the desired angle of entrance and a current angle of entrance and to generate a signal depending on the determined deviation.

9. The system as defined by any of the preceding claims, wherein the tissue is bone, wherein the system (8) further comprises a proximity information providing unit (12) configured to provide proximity information being indicative of a distance between the penetrating device (7) and a cortical wall of the bone (1), wherein the proximity information has been determined based on a measurement carried out at a tip of the penetrating device (7), wherein the model providing unit (10) is configured to provide the model (19) such that it shows the cortical wall of the bone (1), wherein the processor (14) is configured to determine a distance between the penetrating device (7) and the cortical wall based on the provided model (19) and the position of the penetrating device (7) as indicated by the tracking information, to determine a deviation between the distance indicated by the provided proximity information and the determined distance and to determine an accuracy indicator being indicative of the accuracy of the generated virtual view (20) based on the determined deviation.

10. The system as defined by claim 9, wherein the processor (14) is configured to adapt the virtual view (20) by adapting the pose of the penetrating device (7) as indicated by the provided tracking information such that the accuracy indicator indicates an increased accuracy.

11. The system as defined by any of the preceding claims, wherein the system (8) further comprises a tissue information providing unit (13) configured to provide tissue type information about a tissue type as sensed by using the penetrating device (7), wherein the provided model (19) shows different tissue types, wherein the processor (14) is configured to determine an expected tissue type based on the provided model (19) and the provided tracking information, to determine whether the expected tissue type and the tissue type defined by the provided tissue type information match each other and to, if the tissue types do not match, generate a signal indicating the mismatch.

12. The system as defined by any of the preceding claims, wherein the system further comprises a tissue information providing unit (13) configured to provide information about tissue types as sensed by using the penetrating device (7), wherein the processor (14) is configured to generate the virtual view (20) such that it also indicates the sensed tissue types.

13. The system as defined by any of the preceding claims, wherein the model providing unit (10) is configured to provide the model such that it also shows a structure of risk, wherein the processor (14) is configured to determine for multiple regions of the model risk values depending on the distance of the respective region to the structure of risk and to indicate the determined risk values in the multiple regions in the virtual view.

14. Method for assisting a user in placing a penetrating device (7) in tissue, the method (8) comprising:
- receiving or providing tracking information being indicative of a three-dimensional pose of the penetrating device (7),
- receiving or providing a three-dimensional model (19) of the tissue,
- receiving or providing a path (21) through the model (19),
- generating a virtual view (20) from a perspective of a tip of the penetrating device (7) within the tissue in the direction of the received or provided path (21) based on the received or provided tracking information, the received or provided model (19) and the received or provided path (21), wherein the virtual view is generated such that it indicates a direction in which the user should move the penetrating device while placing the penetrating device in the tissue.

15. A computer program for assisting a user in placing a penetrating device in tissue, the computer program comprising program code means for causing a system as defined in any of claims 1 to 13 to carry out the steps of the method as defined in claim 14, when the computer program is run on a computer controlling the system.

16. A computer program as claimed in claim 15 stored on a computer readable medium.
